# EUROPEAN PATENT SPECIFICATION

(11) **EP 2 078 496 B1**
(45) Date of publication and mention of the grant of the patent: **16.05.2012**
(21) Application number: 08425011.7
(22) Date of filing: 09.01.2008
(51) Int. Cl.: A61B 10/02

(54) **Biopsy device and method for the recovery of organic material sampled therewith**
Biopsievorrichtung und Verfahren zur Wiedergewinnung von damit getestetem organischem Material
Dispositif de biopsie et procédé pour la récupération de matière organique prélevé avec ce dispositif

(43) Date of publication of application: 15.07.2009
(73) Proprietor: Zambelli, Roberto, 20017 Rho (MI) (IT)
(72) Inventor: Zambelli, Roberto, 20017 Rho (MI) (IT)
(74) Representative: Petruzziello, Aldo

(56) References cited:
- EP-A- 0 173 653
- GB-A- 2 256 369
- US-A- 5 246 011
- US-A- 5 320 110
- US-A- 5 713 368
- US-B1- 6 413 228

## Description

The present invention pertains to a device for performing a biopsy and to a method for the recovery of the organic material sampled with a biopsy device.

The biopsy consists in sampling small amounts of biological or organic material, from organs such as, for example, the lung, the liver, the kidney, the prostate and the like, in order to examine the material removed. The material sampled is in the form of cells for cytology or of fragments of soft tissues for histology.

In order to perform such biopsies, a special device is used in the form of a syringe comprising a cylinder (syringe body), a piston and a needle cannula (for example a Meneghini needle) with a special tip adapted to penetrate the patient's body until it reaches the organ on which the biopsy is to be performed. Once the needle cannula reaches the organ, the piston of the syringe is withdrawn in order to generate a vacuum in the cylinder chamber and to aspirate the cells of the organ through the needle.

The needle is then removed from the patient's body, the needle tip is placed on a test tube and the piston is pushed forward, with the result of expelling the sampled material into the test tube so that it can be analysed. The main drawback of said biopsy device is precisely that part of the material sampled cannot be expelled because it remains trapped in the needle cannula or even in the aspiration chamber, even when the plunger reaches the end of its stroke. As a result the material sampled at times proves insufficient for adequate analysis.

This drawback is overcome in part by patent EP 0 173 653, in the name of the same applicant, which describes a biopsy device having a stylet or mandrel disposed axially at the end of the plunger to be able to enter the needle cannula. Furthermore, an air valve is provided which puts the aspiration chamber into contact with the outside. Said valve is opened to allow air to be introduced from the outside into the aspiration chamber, and is closed when the piston with the stylet is pushed forward to expel the sampled material from the needle cannula. This operation can be carried out a number of times, until the recovery of the cells trapped in the needle cannula is achieved. In any case, emptying of the device is not efficient and such a device presents the drawback of having to be operated with two hands. In fact, the syringe body is held still with one hand and the piston is operated with the other hand.

To overcome the drawback of the two-handed manoeuvre, semi-automatic biopsy devices provided with a spring disposed between the syringe body and the piston are known to the art. When the piston is pushed forward the spring is loaded and a button that can be operated from the outside locks the piston in position. When the needle cannula reaches the organ on which the biopsy is to be performed, the operator uses the same hand that is gripping the device to press the button that releases the piston, which is retracted through the action of the spring that is released. However, even in this type of semi-automatic device the problem remains of the recovery of the cells that remain trapped in the needle cannula and in the aspiration chamber of the syringe body.

Object of the present invention is to overcome the drawbacks of the prior art by providing a biopsy device and a method of recovering the organic material removed that are reliable, effective, efficient and able to allow the complete recovery of the material removed.

Another object of the present invention is to provide such a device and method that are versatile, and simple for the user to use.

These objects are achieved in accordance with the invention with the device and the method whose characteristics are listed in independent claims 1 and 9, respectively.

Advantageous embodiments of the invention are apparent from the dependent claims.

The biopsy device according to the invention comprises:
- a syringe body defining an aspiration chamber,
- a piston mounted slidably inside the syringe body and provided with a plunger slidable with a tight seal inside the aspiration chamber,
- a needle cannula mounted on the head of the syringe body and provided with a cutting tip adapted to resect the tissue to be sampled,
- a stylet integral with the piston and mounted axially on the piston to slide inside the needle cannula so as to protrude therefrom, said stylet having a percutaneous tip adapted to penetrate the patient's body, until to it reaches the proximity of the organ to be penetrated, and
- a conduit for passage of a liquid, which puts said aspiration chamber into communication with the outside, to inject a liquid into the aspiration chamber through a Luer lock connector made in the syringe body in proximity to the distal end of the aspiration member in order to flush the chamber and the needle cannula so as to extract and to recover the sampled material that has collected therein.

The advantages of the biopsy device according to the invention are evident in that it allows a complete recovery of the sampled organic material, in a way that is efficient and easy for the user.

Further characteristics of the invention will be made clearer by the detailed description that follows, referring to a purely exemplifying and therefore non limiting embodiment thereof, illustrated in the appended drawings, wherein:
Figure 1 is a side view of the biopsy device according to the invention assembled, with the needle cannula shown broken off;
Figure 2 is a side view illustrating the members of the biopsy device of Figure 1 exploded, in which the needle cannula and its connector have been omitted;
Figure 3 is a top plan view of the syringe body of Figure 2;
Figure 4 is an axial sectional view, taken along the plane of section IV-IV of Figure 3;
Figure 5 is an axial sectional view, taken along the plane of section V-V of Figure 4;
Figure 6 is a cross sectional view taken along the plane of section VI-VI of Figure 4;
Figure 7 is a cross sectional view, taken along the plane of section VII-VII of Figure 5;
Figure 8 is a top plan view of the piston of Figure 2, in which the stylet has been omitted;
Figures 9, 10 and 11 are three cross sectional views, enlarged, taken respectively along the planes of section IX-IX, X-X and XI-XI of Figure 8; and
Figure 12 is an enlarged view illustrating two details of the syringe body and of the piston of Figure 2.

The biopsy device according to the invention, designated as a whole with the reference numeral 1, is described with the aid of the figures.

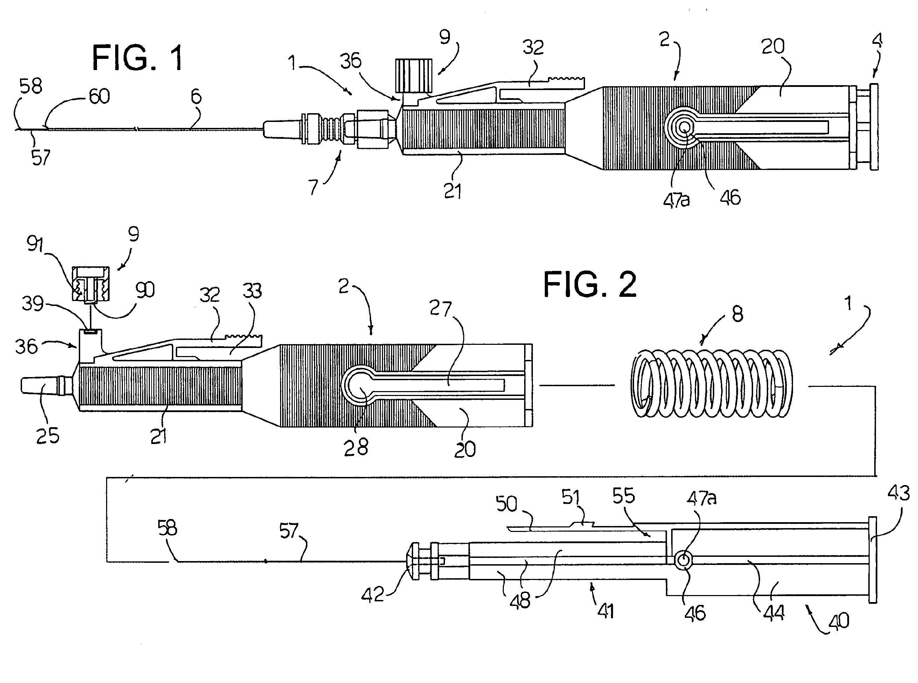

With particular reference for now to Figures 1 and 2, the biopsy device 1 comprises: a syringe body 2, a piston 4, a needle cannula 6, a stylet 57 and a spring 8.

The piston 4 is mounted slidably inside the syringe body 2 and is provided at its end with a plunger 42. The stylet 57 is integral with the piston and protrudes axially from the plunger 42 to slide inside the needle cannula 6. The stylet 57 has a percutaneous tip 58 adapted to penetrate the patient's body until it comes into proximity with the organ to be penetrated.

The needle cannula 6 is provided with a special cutting tip 60 adapted to resect the tissue to be removed. The needle cannula 6 is integral with a needle carrier 7 provided with a special connector to be mounted on the head 25 of the syringe body.

When the piston 4 is at the end of its stroke inside the syringe body 2, the tip 58 of the stylet protrudes slightly with respect to the tip 60 of the needle cannula. In this manner the tip 58 of the stylet acts as a guide during the perforation, until it penetrates the organ, while the tip 60 of the needle cannula resects the tissue. On retracting the piston 2, the tip 58 of the stylet retracts inside the needle cannula 6 and the resected tissue is aspirated into the needle cannula.

Taking the operator as a reference, in the following the term "proximal" indicates the parts of the device near the operator, whereas the term "distal" indicates the parts of the device away from the operator.

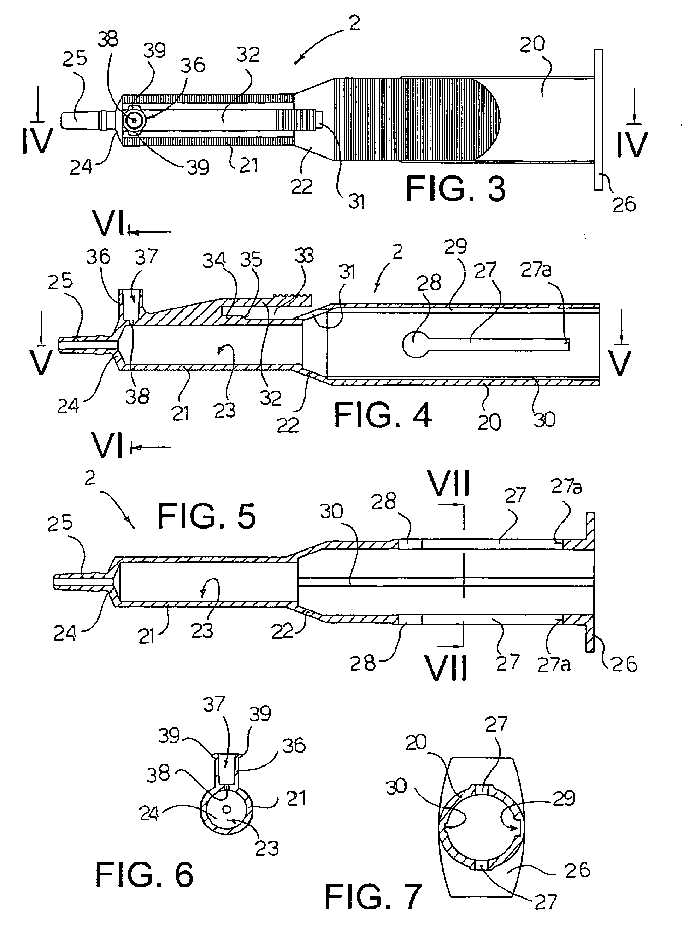

With reference, in particular, to Figures 3-7, the syringe body 2 is hollow on the inside and has a proximal cylindrical portion 20 connected to a distal cylindrical portion 21 with a smaller diameter, by means of an intermediate transitional portion 22 with a tapering shape. Inside the distal cylindrical portion 21 with a smaller diameter an aspiration chamber 23 is defined, into which part of the biological material sampled during the biopsy could be aspirated.

The distal cylindrical portion 21 ends with a shoulder 24 which proceeds with a truncated conical head 25 with a smaller diameter adapted to engage with the connector of the needle-carrier 7, per se known and therefore not described in detail.

A radial flange 26 with two side tongues, acting as a rest for the user's fingers, is provided at the proximal end of the proximal cylindrical portion 20.

Two longitudinal slots 27 disposed in diametrically opposite positions are formed in the proximal cylindrical portion 20. Each longitudinal slot 27 is delimited by an abutment surface 27a and ends in a circular hole 28.

Two longitudinal channels 29 and 30 disposed in diametrically opposite positions and spaced apart 90° by longitudinal slots 27 are formed in the inner surface of the proximal cylindrical portion 20. The longitudinal channel 29 is wider and deeper than the longitudinal channel 30. The two longitudinal channels 29 and 30 extend for the whole length of the proximal cylindrical portion 20. The widest channel 29 opens into a slot 31 formed in the median transitional portion 22.

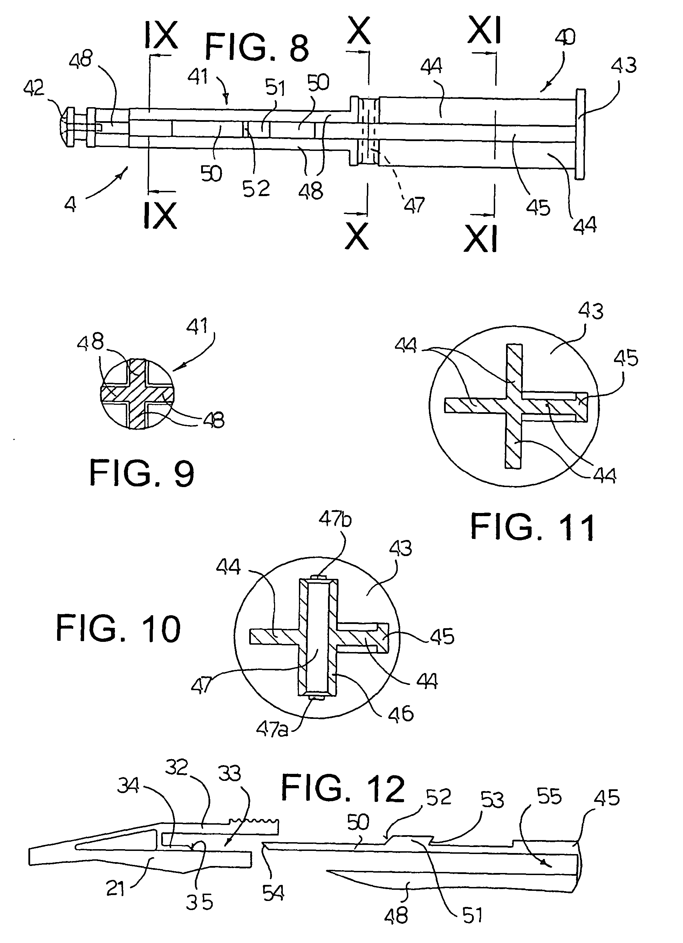

A flexible longitudinal tongue 32, which extends over the slot 31 of the median transitional portion 22, is formed on the distal cylindrical portion 21. A gap 33 is left between the flexible tongue 32 and the outer surface of the distal cylindrical portion 21. As shown better in Figure 12, the distal cylindrical portion has a protrusion 34 beneath the flexible tongue 32, in the gap 33. The protrusion 34 has a tapered entry surface 35.

A connector 36, which extends radially outwards, is formed in the distal cylindrical portion 21, near the shoulder 24. The connector 36 defines a radial conduit 37 communicating with a radial hole 38 formed in the distal portion 21 near the distal end of the aspiration chamber 23. In this manner the radial conduit 37 is in communication with the distal end of the aspiration chamber 23. The hole 38 has a diameter of about 1 mm to allow a liquid under pressure to be introduced easily from the outside into the aspiration chamber 23.

The connector 36 is made according to the Luer lock criteria. Therefore, the radial conduit 37 has a substantially truncated conical profile to allow a conical coupling with a matching male connector. Furthermore, two radial tongues 39, which protrude in diametrically opposite directions, adapted to engage in a thread, are formed at the end of the connector 36. In this manner the connector 36 can be coupled with the head of a standard syringe or with any other type of medical instrument provided with a Luer lock type connector.

The connector 36 is closed with a airtight seal, by means of a stopper 9 (Figures 1 and 2) provided with an axial truncated conical tang 90 which is inserted into the conduit 37 of the connector and an inside thread 91 which is screwed onto the tongues 39 of the connector.

With reference to Figures 2 and 8 to 11, the piston 4 comprises a stem, having a cross-shaped cross section, formed by a proximal portion 40 and by a distal portion 41 ending in a plunger 42 adapted to slide air-tightly inside the aspiration chamber 23 of the syringe body. At the proximal end of the proximal portion 40 there is a circular flange 43 acting as a resting surface for the user's thumb to push the piston 4 forward.

The proximal portion 40 has four stiff ribs 44 disposed in the form of a "+" in cross section, so as to define a diameter adapted to slide inside the proximal portion 20 of the syringe body. One of the ribs 44 has at the end a rib 45 with a greater width, so as to be substantially T-shaped in section. The rib with a greater width 45 is adapted to slide in the channel 29 with a greater width of the proximal portion 20 of the syringe body. The rib 44, diametrically opposite the rib 45, is of such a thickness as to be able to slide in the channel 30 with a smaller width of the proximal portion 20 of the syringe body.

Near the distal portion 41 of the stem, a cylindrical tang 46, which extends according to the diameter of the stem, is integrated within two diametrically opposite ribs 44. A pin 47 having two ends 47a and 47b protruding from the tang 46 and acting as guide pins or nibs is inserted into the tang 46. The ends 47a and 47b of the pin 47 are adapted to slide in the longitudinal channels 27 of the proximal portion of the syringe body.

The pin 47 is coupled forcibly in the tang 46, after which the piston is inserted into the syringe body and the tang 46 is situated level with the holes 28 at the end of the longitudinal slots 27. Otherwise, if the pin 47 is pre-mounted in the piston 4, the piston must be inserted forcibly into the syringe body, so that the protrusions 47a and 47b of the pin 47 are inserted into the channels 27 of the syringe body. Once the piston has been mounted inside the syringe body, it can no longer be extracted, since the nibs 47a and 47b abut against the end-of-stroke walls 27a of the slots 27.

The distal part 41 of the piston stem has four ribs 48 crossed in a "+" shape in cross section, with a diameter adapted to slide inside the aspiration chamber 23 of the distal portion of the syringe body.

The protruding part 45 of the rib 44 of the proximal portion of the stem continues in a flexible tongue 50, which extends above a rib 48 of the distal part of the stem, forming a gap 55 between the flexible tongue 50 and the rib 48. As shown better in Figure 12, on the flexible tongue 50 there is a protruding part 51 which defines a tapered entry surface 52 with an acute angle and a tapered exit surface 53 with an obtuse angle. The end 54 of the flexible tongue 50 is tapered. The flexible tongue 50 is adapted to be inserted in the slot 31 formed in the median transitional portion 2 of the syringe body.

The spring 8 is of the spiral type and is adapted to be disposed around the distal portion 41 of the piston stem, entering the gap 55 formed by the elastic tongue 50 of the stem. Furthermore, the spring 8 is also inserted into the proximal portion 20 of the syringe body. In this manner, one end of the spring 8 abuts against the end of the ribs 44 and of the tang 46 of the proximal portion 40 of the stem and the other end of the spring 8 abuts against the narrowing that forms the median transitional portion 22 of the syringe body.

Operation of the biopsy device according to the invention is described below.

The user initially pushes the piston 4 forwards by pressing with his or her thumb on the proximal flange 43 of the piston and by holding the proximal flange 26 of the syringe body with the index and middle finger. As a result, the spring 8 is compressed and loaded. The flexible tongue 50 of the piston passes through the slot 31 in the tapered median part 22 of the syringe body disposing itself in the gap 33 beneath the flexible tongue 32 of the syringe body. The stylet 57 slides inside the needle cannula 60 so that the tip 58 of the stylet exits from the tip 60 of the needle cannula.

During the forward movement of the piston, the tapered entry surface 52 of the protrusion 51 of the piston tongue slides beneath the edge of the slot 31, bending the tongue 50 in the gap 55; at the same time the tapered end 54 of the tongue 50 slides on the tapered entry surface 35 of the protrusion 34 beneath the rib 32, thus raising the rib 50. When the protrusion 51 goes past the slot 31, the tongue 50 returns elastically into the starting position and the tapered exit surface 53 of the protrusion 51 abuts against the edge of the slot 31, thus retaining the piston 4 in its forward position against the action of the spring 8. In this situation the plunger 42 is at the end of its stroke in the aspiration chamber 23 and the spring 8 is loaded under compression.

Subsequently, the user ensures that the stopper 9 is screwed to the connector 36 to close the conduit 37 tightly and inserts the tip 58 of the stylet into the patient's skin. The tip 58 of the stylet acts as a guide for the tip 60 of the needle cannula 6 allowing the needle cannula 6 to be inserted until the tip 58 of the stylet perforates the organ on which the biopsy is to be done.

At this point the user presses the flexible tongue 32 of the syringe body. As a result, the flexible tongue 32 of the syringe body presses the protrusion 51 of the flexible tongue 50 of the piston, until the tapered exit surface 53 of the protrusion 51 no longer interferes with the edge of the slot 31 and thus the piston is no longer held in place. Consequently, through the action of the spring 8, which is released, the piston 4 is pushed backwards, until the pins 47a and 47b of the piston abut against the abutment surfaces 27a and 27b of the longitudinal slots 27 of the syringe body. It should be noted that the backward movement of the piston 4, besides being guided by the sliding of the pins 47a and 47b of the piston in the respective slots 27 of the syringe body, is also guided by the sliding of the ribs 44 and 45 of the proximal part of the piston inside the channels 30 and 29 of the proximal part of the syringe body.

During the backward movement of the plunger 42, a vacuum is created in the aspiration chamber 23. The retraction of the piston 4 also causes a retraction of the stylet 57 inside the needle cannula 6. The operator causes the needle to make a short excursion into the organ to be biopsied. As a result, the cells and the fragments of the organ resected by the tip 60 of the needle cannula are aspirated into the needle cannula 6, into the space freed by the stylet 57, and also in part into the aspiration chamber 23.

At this point the user extracts the needle cannula 6 from the patient and places the tip 60 of the needle cannula in a test tube for collecting the material sampled, then pushes the piston 4 forward. Consequently the stylet 57 slides forward inside the needle cannula 6 to push the sampled material out of the needle cannula 6 into the test tube.

Subsequently, the user opens the stopper 9 and connects the connector 36 to a fluid supply source. Said fluid supply source can be a standard syringe provided with a tip adapted to couple with the connector 36 or can be any other medical device provided with a Luer type connector.

At this point, a fluid suitable for biopsy analyses, such as for example the Saccomanno's fluid, is made to flow in through the conduit 37. This fluid passes through the hole 38 and enters the aspiration chamber 23, to flow outward through the needle cannula 6 and the conduit 37. The fluid performs a perfect flushing of the aspiration chamber 23 and of the inside of the needle cannula 6, carrying with it all the sampled cells and fragments.

Numerous changes and modifications of detail within the reach of a person skilled in the art can be made to the present embodiment of the invention without thereby departing from the scope of the invention as set forth in the appended claims.

## Claims

1. A biopsy device (1) comprising:
- a syringe body (2) defining an aspiration chamber (23),
- a piston (4) mounted slidably inside the syringe body and provided with a plunger (42) tightly slidable inside the aspiration chamber (23),
- a needle cannula (6) mounted on the head (25) of the syringe body (2) and provided with a cutting tip (60) adapted to resect the tissue to be removed,
- a stylet (57) integral with the piston and mounted axially on the plunger (42) to slide inside the needle cannula (6) so as to protrude therefrom, said stylet having a percutaneous tip (58) adapted to penetrate the patient's body until it arrives in proximity to the organ to be penetrated,
- a conduit (37) made in the syringe body (2) in proximity to the distal end of the aspiration chamber (23) for the passage of a liquid, which puts said aspiration chamber (23) into communication with the outside, **characterised in that** said conduit (37) is formed in a connector (36) of the Luer lock type for a syringe or a medical instrument, through which a liquid can be injected into the aspiration chamber in order to flush the chamber and the needle cannula (6) so as to extract and to recover the sampled material.

2. A device (1) according to claim 1, **characterised in that** said connector (36) protrudes radially from the syringe body.

3. A device (1) according to claim 1 or 2, **characterised in that** said conduit (37) has a truncated conical shape.

4. A device (1) according to any one of the preceding claims, **characterised in that** it comprises a removable stopper (9) adapted to close said conduit (37) with a tight seal.

5. A device (1) according to any one of the preceding claims, **characterised in that** it comprises:
- a spring (8) interposed between an abutment surface of the syringe body (2) and an abutment surface of the piston (4),
- locking means (50) adapted to lock the piston in a forward position in which the spring (8) is compressed;
- release means (32) that can be operated manually by the user to release the locking means (50), so that the piston is pushed backward by the spring (8), creating the vacuum in the aspiration chamber (23).

6. A device (1) according to claim 5, **characterised in that**:
- said locking means (50) comprise a longitudinal flexible tongue (50) formed on the piston, adapted to come out of the syringe body through a slot (31) of the syringe body to lock on the edge of the slot (31), and
- said release means (32) comprise a longitudinal flexible tongue (32) formed on the syringe body, adapted to compress said flexible tongue (50) of the piston to disengage it from the edge of the slot (31).

7. A device (1) according to any one of the preceding claims, **characterised in that** it comprises:
- guide means (29, 30, 27, 45, 44, 47a, 47b) to guide the compression and the aspiration stroke of the piston (4) inside the syringe body (2), and
- end of stroke means (27a, 27b; 47a, 47b) to lock the aspiration stroke of the piston, so as to prevent it from coming out of the syringe body.

8. A device (1) according to claim 7, **characterised in that**
- said guide means comprise longitudinal channels (29, 30) and longitudinal slots (27) formed in a proximal cylindrical portion (20) of the syringe body within which can slide ribs (45, 44) and pins (47a, 47b) formed in a proximal portion (40) of the piston stem, and
- said longitudinal slots (27) have a proximal abutment surface (27a) acting as a stroke-limiting stop to block said pins (47a, 47b) of the piston stem.

9. A method for recovering organic material sampled by a biopsy device (1) comprising:
- a syringe body (2) defining an aspiration chamber (23),
- a piston (4) mounted slidably inside the syringe body and provided with a plunger (42) tightly slidable inside the aspiration chamber (23),
- a needle cannula (6) mounted on the head (25) of the syringe body (2) and provided with a cutting tip (60) adapted to resect the tissue to be sampled,
- a stylet (57) integral with the piston and mounted axially on the plunger (42) to slide inside the needle cannula (6) so as to protrude therefrom, said stylet having a percutaneous tip (58) adapted to penetrate the patient's body until it arrives in proximity to the organ to be penetrated,
**characterised in that**
- said method comprises the step of injecting a liquid into the aspiration chamber (23) through a Luer lock connector (36) in order to flush the aspiration chamber (23) and the needle cannula (6) so as to extract and to recover the sampled material.

10. A method according to claim 9, **characterised in that** said fluid is injected by means of a syringe that has a head adapted to couple with said Luer lock connector (36) of said device (1) defining a conduit (37) communicating with said aspiration chamber (23).

## Patentansprüche

1. Biopsievorrichtung (1), welche Folgendes aufweist:
- einen Spritzenkörper (2), der eine Aspirationskammer (23) definiert,
- einen Kolben (4), der verschiebbar innerhalb des Spritzenkörpers befestigt ist und der mit einem Plunger (42) versehen ist, der innerhalb der Aspirationskammer (23) eng verschiebbar ist,
- eine Nadelkanüle (6), die am Kopf (25) des Spritzenkörpers (2) befestigt ist und die mit einer Schneidspitze (60) versehen ist, die zum Herausschneiden des zu entnehmenden Gewebes geeignet ist,
- ein Stilett (57), das in den Kolben integriert ist und das axial an dem Plunger (42) befestigt ist, um innerhalb der Nadelkanüle (6) zu gleiten, so dass es daraus hervorsteht, wobei das Stilett eine perkutane Spitze (58) aufweist, die zum Penetrieren des Körpers des Patienten geeignet ist, bis sie in die Nähe des zu penetrierenden Organs gelangt,
- einen Kanal (37) gebildet im Spritzenkörper (2) in der Nähe des distalen Endes der Aspirationskammer (23) für den Durchgang einer Flüssigkeit, welcher die Aspirationskammer (23) mit der äußeren Umgebung verbindet, **dadurch gekennzeichnet, dass** der Kanal (37) in einem Verbindungsstück (36) des Luer-Lock-Typs für eine Spritze oder ein medizinisches Instrument ausgebildet ist, durch welchen eine Flüssigkeit in die Aspirationskatnmer injiziert werden kann, um die Kammer und die Nadelkanüle (6) zu spülen und so das Probenmaterial zu extrahieren und wiederzugewinnen.

2. Vorrichtung (1) nach Anspruch 1, **dadurch gekennzeichnet, dass** das Verbindungsstück (36) radial von dem Spritzenkörper absteht.

3. Vorrichtung (1) nach Anspruch 1 oder 2, **dadurch gekennzeichnet, dass** der Kanal (37) eine abgeschnittene konische Form aufweist.

4. Vorrichtung (1) nach einem der vorhergehenden Ansprüche, **dadurch gekennzeichnet, dass** sie einen entfernbaren Stopper (9) aufweist, der zum Verschließen des Kanals (37) mit einer engen Abdichtung geeignet ist.

5. Vorrichtung (1) nach einem der vorhergehenden Ansprüche, **dadurch gekennzeichnet, dass** sie Folgendes aufweist:
- eine Feder (8) eingefügt zwischen einer Anlagefläche des Spritzenkörpers (2) und einer Anlagefläche des Kolbens (4),
- ein Verriegelungsmittel (50), das zum Verriegeln des Kolbens in einer Vorwärtsposition, in welcher die Feder (8) komprimiert ist, geeignet ist,
- ein Freigabemittel (32), das manuell durch den Benutzer betätigt werden kann, um das Verriegelungsmittel (50) freizugeben, so dass der Kolben durch die Feder (8) zurückgedrückt wird, wodurch das Vakuum in der Aspirationskammer (23) erzeugt wird.

6. Vorrichtung (1) nach Anspruch 5, **dadurch gekennzeichnet, dass**:
- das Verriegelungsmittel (50) eine flexible Zunge in Längsrichtung (50) aufweist, die an dem Kolben ausgebildet ist, welche dazu geeignet ist, durch einen Schlitz (31) des Spritzenkörpers aus dem Spritzenkörper herauszukommen, um an der Kante des Schlitzes (31) zu arretieren, und
- das Freigabemittel (32) eine flexible Zunge in Längsrichtung (32) aufweist, die an dem Spritzenkörper ausgebildet ist, welche dazu geeignet ist, die flexible Zunge (50) des Kolbens zu komprimieren, um diese von der Kante des Schlitzes (31) zu lösen.

7. Vorrichtung (1) nach einem der vorhergehenden Ansprüche, **dadurch gekennzeichnet, dass** sie Folgendes aufweist:
- Führungsmittel (29, 30, 27, 45, 44, 47a, 47b) zum Führen der Kompression und des Aspirationshubs des Kolbens (4) innerhalb des Spritzenkörpers (2), und
- Hubendmittel (27a, 27b; 47a, 47b) zum Verriegeln des Aspirationshubs des Kolbens um zu verhindern, dass er aus dem Spritzenkörper herauskommt.

8. Vorrichtung (1) nach Anspruch 7, **dadurch gekennzeichnet, dass**:
- die Führungsmittel Längskanäle (29, 30) und Längsschlitze (27) ausgebildet in einem proximalen zylindrischen Abschnitt (20) des Spritzenkörpers aufweisen, innerhalb welcher Rippen (45, 44) und Stifte (47a, 47b) ausgebildet in einem proximalen Abschnitt (40) des Kolbenschaftes gleiten können, und
- die Längsschlitze (27) eine proximale Anlagefläche (27a) aufweisen, die als ein Hub-begrenzender Stopp zum Blockieren der Stifte (47a, 47b) des Kolbenschaftes agiert.

9. Verfahren zur Wiedergewinnung von mit einer Biopsievorrichtung (1) entnommenem organischem Material, welche Folgendes aufweist:
- einen Spritzenkörper (2), der eine Aspirationskammer (23) definiert,
- einen Kolben (4), der verschiebbar innerhalb des Spritzenkörpers befestigt ist und der mit einem Plunger (42) versehen ist, der innerhalb der Aspirationskammer (23) eng verschiebbar ist,
- eine Nadelkanüle (6), die am Kopf (25) des Spritzenkörpers (2) befestigt ist und die mit einer Schneidspitze (60) versehen ist, die zum Herausschneiden des zu entnehmenden Gewebes geeignet ist,
- ein Stilett (57), das in den Kolben integriert ist und das axial an dem Plunger (42) befestigt ist, um innerhalb der Nadelkanüle (6) zu gleiten, so dass es daraus hervorsteht, wobei das Stilett eine perkutane Spitze (58) aufweist, die zum Penetrieren des Körpers des Patienten geeignet ist, bis sie in die Nähe des zu penetrierenden Organs gelangt,
**dadurch gekennzeichnet, dass**
- das Verfahren den Schritt des Injizierens einer Flüssigkeit in die Aspirationskammer (23) durch ein Luer-Lock-Verbindungsmittel (36) zum Spülen der Aspirationskammer (23) und der Nadelkanüle (6) zum Extrahieren und Wiedergewinnen des entnommenen Materials umfasst.

10. Verfahren nach Anspruch 9, **dadurch gekennzeichnet, dass** das Fluid mit Hilfe einer Spritze injiziert wird, welche einen Kopf aufweist, der zum Koppeln mit dem Luer-Lock-Verbindungsmittel (36) der Vorrichtung (1) geeignet ist, welches einen Kanal (37) in Verbindung mit der Aspirationskammer (23) definiert.

## Revendications

1. Dispositif de biopsie (1) comprenant :
- un corps de seringue (2) définissant une chambre d'aspiration (23),
- un piston (4) monté de manière coulissante à l'intérieur du corps de seringue et doté d'un plongeur (42) coulissant de manière serrée à l'intérieur de la chambre d'aspiration (23),
- une canule d'aiguille (6) montée sur la tête (25) du corps de seringue (2) et dotée d'une extrémité tranchante (60) adaptée à couper le tissu à prélever,
- un stylet (57) formé en une seule pièce avec le piston et monté axialement sur le plongeur (42) pour coulisser à l'intérieur de la canule d'aiguille (6) de façon à dépasser de ladite canule, ledit stylet ayant une extrémité percutanée (58) adaptée à pénétrer dans le corps du patient jusqu'à ce qu'elle arrive à proximité de l'organe à pénétrer,
- un conduit (37) formé dans le corps de seringue (2) à proximité de l'extrémité distale de la chambre d'aspiration (23) pour le passage d'un liquide qui met ladite chambre d'aspiration (23) en communication avec l'extérieur, **caractérisé en ce que** ledit conduit (37) est formé dans un raccord (36) de type Luer lock pour une seringue ou un instrument médical, par lequel un liquide peut être injecté dans la chambre d'aspiration de manière à rincer la chambre et la canule d'aiguille (6) afin d'extraire et de récupérer la matière prélevée.

2. Dispositif (1) selon la revendication 1, **caractérisé en ce que** ledit raccord (36) dépasse radialement du corps de seringue.

3. Dispositif (1) selon la revendication 1 ou 2, **caractérisé en ce que** ledit conduit (37) a une forme conique tronquée.

4. Dispositif (1) selon l'une quelconque des revendications précédentes, **caractérisé en ce qu'**il comprend un bouchon amovible (9) adapté à fermer ledit conduit (37) avec un joint étanche.

5. Dispositif (1) selon l'une quelconque des revendications précédentes, **caractérisé en ce qu'**il comprend :
- un ressort (8) interposé entre une surface de butée du corps de seringue (2) et une surface de butée du piston (4),
- un moyen de blocage (50) adapté à bloquer le piston dans une position avant dans laquelle le ressort (8) est compressé ;
- un moyen de libération (32) qui peut être actionné manuellement par l'utilisateur pour libérer le moyen de blocage (50), de sorte que le piston est poussé vers l'arrière par le ressort (8), créant le vide dans la chambre d'aspiration (23).

6. Dispositif (1) selon la revendication 5, **caractérisé en ce que** :
- ledit moyen de blocage (50) comprend une longuette longitudinale flexible (50) formée sur le piston, adaptée à sortir du corps de seringue à travers une fente (31) dans le corps de seringue pour se bloquer sur le bord de la fente (31), et
- ledit moyen de libération (32) comprend une languette longitudinale flexible (32) formée sur le corps de seringue, adaptée à compresser ladite languette flexible (50) du piston pour la libérer du bord de la fente (31).

7. Dispositif (1) selon l'une quelconque des revendications précédentes, **caractérisé en ce qu'**il comprend :
- un moyen de guidage (29, 30, 27, 45, 44, 47a, 47b) pour guider la compression et la course d'aspiration du piston (4) à l'intérieur du corps de seringue (2), et
- un moyen de fin de course (27a, 27b ; 47a, 47b) pour bloquer la course d'aspiration du piston, afin de l'empêcher de sortir du corps de seringue.

8. Dispositif (1) selon la revendication 7, **caractérisé en ce que**
- ledit moyen de guidage comprend des canaux longitudinaux (29, 30) et des fentes longitudinales (27) formées dans la partie cylindrique proximale (20) du corps de seringue à l'intérieur duquel peuvent coulisser des nervures (45, 44) et des broches (47a, 47b) formées dans une partie proximale (40) de la tige de piston, et
- lesdites fentes longitudinales (27) ont une surface de butée proximale (27a) servant de butée de limitation de course pour bloquer lesdites goupilles (47a, 47b) de la tige de piston.

9. Procédé de récupération de matière organique prélevée par un dispositif de biopsie (1), qui comprend :
- un corps de seringue (2) définissant une chambre d'aspiration (23),
- un piston (4) monté de manière coulissante à l'intérieur du corps de seringue et doté d'un plongeur (42) coulissant de manière serrée à l'intérieur de la chambre d'aspiration (23),
- une canule d'aiguille (6) montée sur la tête (25) du corps de seringue (2) et dotée d'une extrémité tranchante (60) adaptée à couper le tissu à prélever,
- un stylet (57) formé en une seule pièce avec le piston et monté axialement sur le plongeur (42) pour coulisser à l'intérieur de la canule d'aiguille (6) de façon à dépasser de ladite canule, ledit stylet ayant une extrémité percutanée (58) adaptée à pénétrer dans le corps du patient jusqu'à ce qu'elle arrive à proximité de l'organe à pénétrer,
**caractérisé en ce que**
- ledit procédé comprend l'étape consistant à injecter un liquide dans la chambre d'aspiration (23) par un raccord Luer lock (36) de manière à rincer la chambre d'aspiration (23) et la canule d'aiguille (6) afin d'extraire et de récupérer la matière prélevée.

10. Procédé selon la revendication 9, **caractérisé en ce que** ledit fluide est injecté au moyen d'une seringue qui a une tête adaptée à se coupler au dit raccord Luer lock (36) dudit dispositif (1) définissant un conduit (37) en communication avec ladite chambre d'aspiration (23).
